# EUROPEAN PATENT APPLICATION

(11) **EP 3 231 382 A1**
(43) Date of publication of application: **18.10.2017**
(21) Application number: 16382168.9
(22) Date of filing: 13.04.2016
(51) Int. Cl.: A61B 17/70

(54) **DYNAMIC APPARATUS FOR SPINAL DISK TRACTION**

(71) Applicant: Morales, José, 07469 Mexico (MX)
(72) Inventor: Morales, José, 07469 Mexico (MX)
(74) Representative: Urizar Anasagasti, Jesus Maria

(57) **Abstract**

An apparatus for treating spinal vertebrae of patients that require therapeutic mechanical effects. Two interconnected articulated U-shape frame assemblies **(20, 70)** provide the necessary supporting at adjustable relative angular disposition with respect to each other to conform with the characteristics of the spinal segment being treated. Upper frame assembly (**20)** is mounted at a predetermined location of a patient's spine. Connector assemblies (**40, 40')** are mounted to upper assembly (**20)** and to telescopic tubular assemblies (**50, 50')**. Pivoting connectors **(60, 60')** are connected to the tubular assemblies (**50, 50')** and provide pivoting movement in two orthogonal planes for tubular assemblies (**50, 50')** and lower frame assembly (**70).**

## Description

### BACKGROUND OF THE INVENTION

### Field of the Invention.

The present invention relates to an orthopedic surgical apparatus for treating spinal deformities and, more particularly, to a articulated apparatus for disk traction.

### Description of the Related Art.

Several apparatuses for treating deformities have been designed in the past. None of them, however, include the articulation characteristics of the present invention providing ergonometric movements that track those naturally found in spinal segments of a patient.

Applicant believes that the closest reference corresponds to U.S. patent No. 5,810,815 issued to Applicant in 1998. However, it differs from the present invention because Applicant's patented surgical apparatus is not capable of dynamically follow the natural movements of a user's spine. The rigidity of the patented apparatus limits the movements allowed. These limitations are overcome with Applicant's new articulated frame apparatus for disk traction.

Other documents describing the closest subject matter provide for a number of more or less complicated features that fail to solve the problem in an efficient and economical way. None of these patents suggest the novel features of the present invention.

### SUMMARY OF THE INVENTION

It is one of the main objects of the present invention to provide an orthopedic surgical apparatus that is used for segmental fixation of vertebrae in the spinal column.

It is another object of this invention to provide such an ergonomical apparatus that can be readily adapted to the characteristics of a user's spine providing the necessary rigidity while allowing predetermined movement tracking the spine's natural positions.

It is still another object of the present invention to provide an orthopedic surgical apparatus that can be readily mounted to a patient's body.

It is yet another object of this invention to provide such an apparatus that is inexpensive to manufacture and maintain while retaining its effectiveness.

Further objects of the invention will be brought out in the following part of the specification, wherein detailed description is for the purpose of fully disclosing the invention without placing limitations thereon.

### BRIEF DESCRIPTION OF THE DRAWINGS

With the above and other related objects in view, the invention consists in the details of construction and combination of parts as will be more ully understood from the following description, when read in conjunction with the accompanying drawings in which:
**Figure 1** represents an exploded isometric view showing the different components of an embodiment for the disk traction apparatus disclosed in the present application prior to being assembled.
**Figure 2** shows an elevational front view of the embodiment represented in the previous figure.
**Figure 2A** illustrates an elevational cross-sectional view taken along line A-A' in figure 2.
**Figure 2B** is an enlarged cross-sectional view of a connector assembly **40** mounted to threaded end **25** and tubular portion **53** partially shown.
**Figure 3** is a rear elevational view of the device shown in figure 2.
**Figure 3A** is an enlarged isometric sectional representation of portions of tubular assembly **50** and lower assembly **70**, both pivotally mounted to pivoting connector assembly **60**.
**Figure 3B** is a longitudinal cross-sectional isometric representation of a portion of tubular assembly **50** slidably mounted threaded rod portion **25** and cylindrical portion **45**.
**Figure 3C** is an enlarged cross representation of pivoting connector assembly **60** with riveted pins **80**.
**Figure 3D** is a longitudinal cross-sectional isometric representation of tubular assembly **50**.
**Figure 3E** is a longitudinal cross-sectional isometric representation of assembly **50**.
**Figure 3F** is an end view of connector assembly **40**.
**Figure 3G** is a longitudinal cross-sectional isometric representation of assembly **40**.
**Figure 4** shows a side elevational view of the embodiment of the apparatus represented in the previous figures in three different positions (one with solid lines and the other one two broken lines) illustrating its dynamic orthopedic capabilities.
**Figure 5** shows an elevational front view of the embodiment of the apparatus shown in the previous figures pivoted to the sides.

### DETAILED DESCRIPTION OF THE EMBODIMENTS OF THE INVENTION

Referring now to the drawings, where the present invention is generally referred to with numeral **10**, it can be observed that it basically includes upper frame assembly **20** having substantially a U-shape, mounted to connector assemblies **40**; **40'** that in turn are mounted to frame assembly **20** with fastening members **90**; **90'**. Tubular assemblies **50**; **50'** are slidably mounted to assemblies **20** and **40**. Tubular assemblies **50**; **50'** are pivotally (side movements) mounted to pivoting connector assemblies **60; 60'** which are also pivotally (front-rear movements) connected to lower frame assembly **70**.

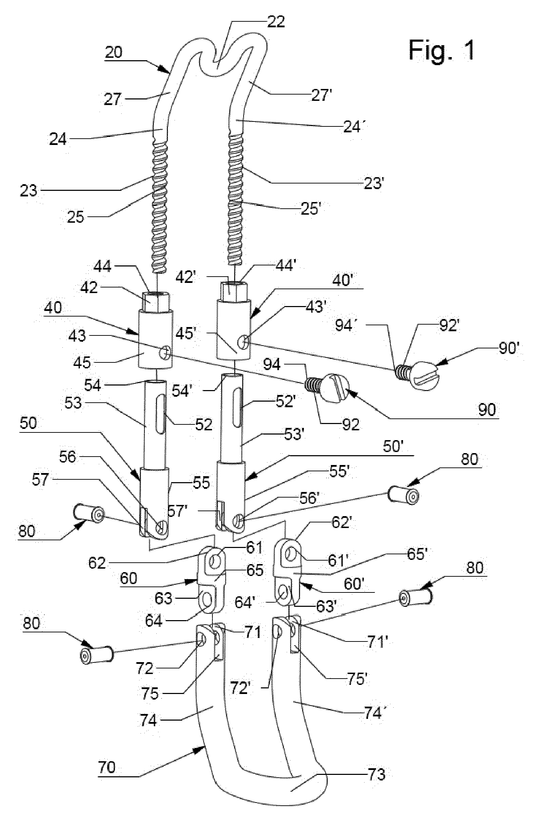

Upper frame assembly **20** with elongated bars **23** and **23'** each have straight non-threaded portions **24; 24',** straight threaded portions **25; 25',** and angled portions **27; 27'**, respectively, as best seen in figure 1. Assembly **20** has substantially U-shape configuration. Spacer member **22** is rigidly mounted to the upper ends of portions **27**; **27'** keeping bars **23**; **23'** at a parallel and spaced apart relationship with respect to each other. Spacer member **22** has a cooperating ergonomic shape compatible with the bone structure where it is going to be affixed.

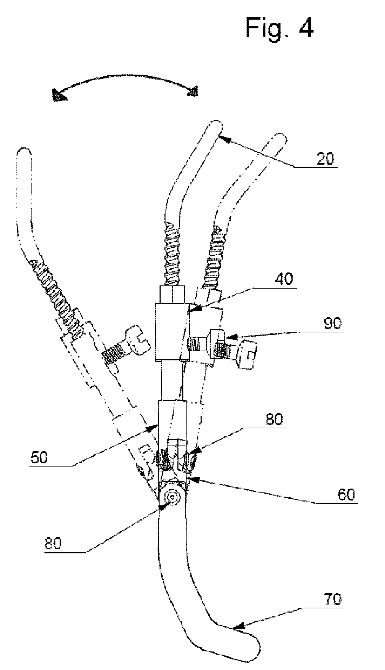

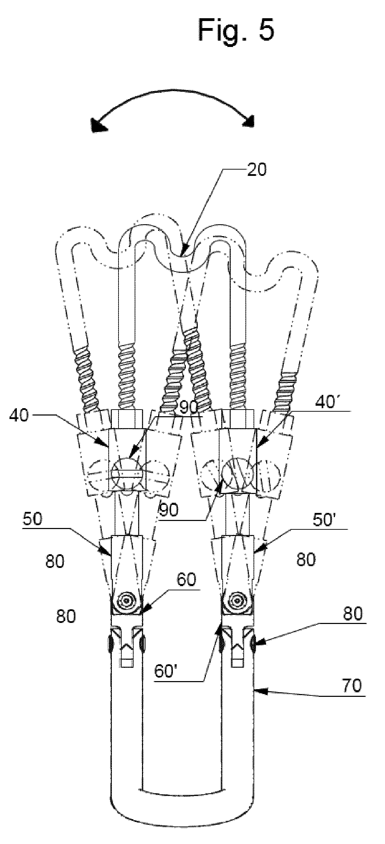

For the purpose of this application, Applicant will refer to "lateral" or "sides" to positions or movements to the right or left of apparatus **10** from an upright elevational view as seen in figure 5. Whereas front and rear positions or movements will be those shown in figure 4.

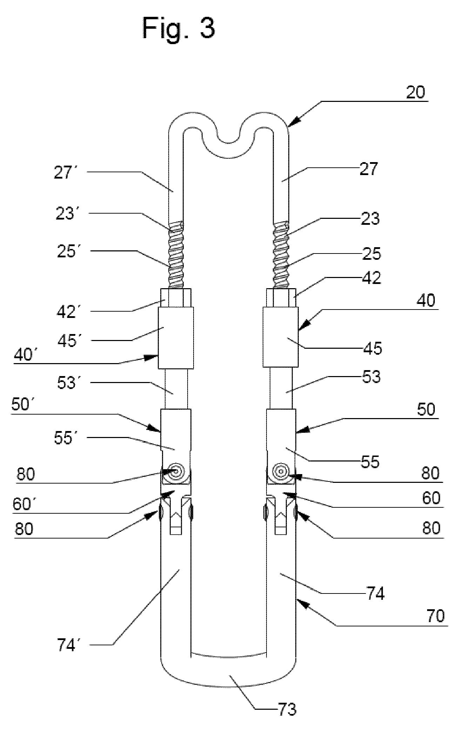

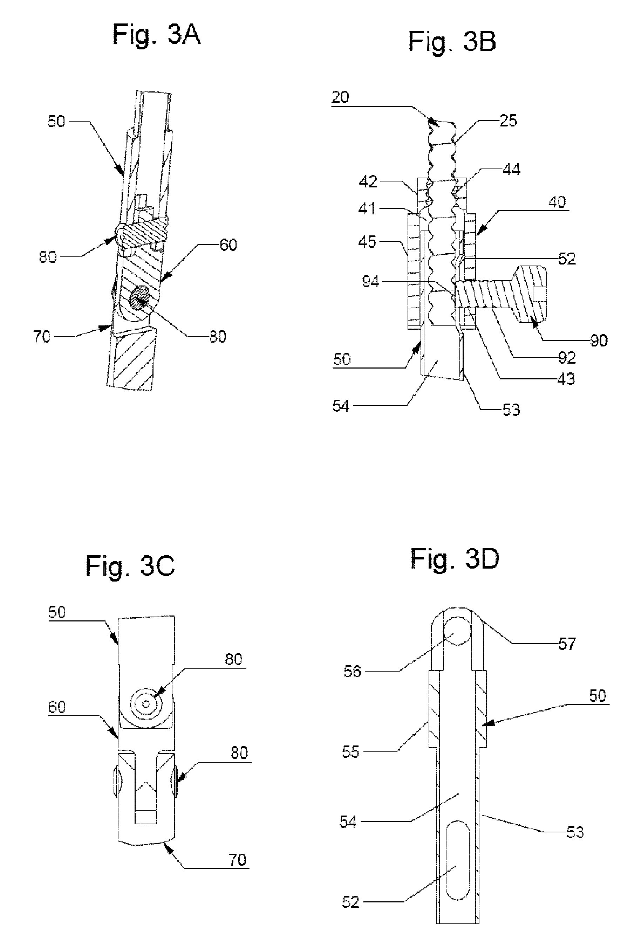

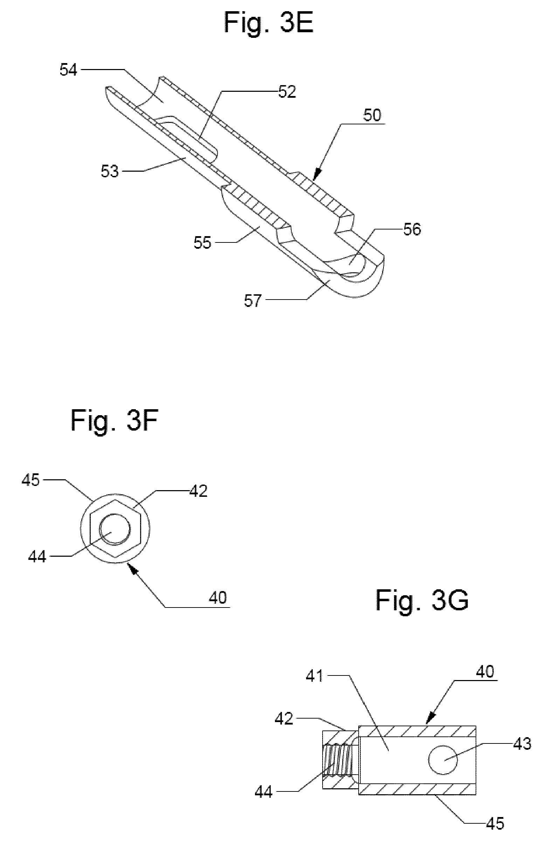

Connector assemblies **40; 40'** have an elongated shape with cylindrical portions **45; 45'** at one end and polygonal portions **42; 42'**, at the other end, as best seen in figures 1, 3F and 3G. Portion **45**, has coaxial through opening **41** (only shown for portion **45**, but portion **45'** includes a similar coaxial through opening), as seen in figure 3G. Threaded through openings **43**; **43'** engage with fasteners **90**; **90'** that pass through one side of portions **45**; **45'** and through slots **52**; **52'** to exert a pressure against threaded bar **23; 23';** respectively. Portion **42** has coaxially disposed threaded through opening **44** (only shown for portion **42** but portion **42'** includes a similar threaded through opening) for cooperative receiving threaded portions **25**; **25'**. Through opening **41** for connector assembly **40** (and similarly for **40'**) has an inner diameter that is larger that the inner diameter of threaded through opening **44** to allow a predetermined sliding movement of portion **53** (and **53'**), as seen in figure 3B.

Polygonal portions **42**; **42'** can be readily actuated (rotated) using a cooperating wrench. Threaded through opening **44**; **44'** mate with threaded rod portions **25**; **25'** to advance the former along the latter securing a firm telescopic (adjustable to the needs of each patient) engagement between connector assemblies **40**; **40'** and upper frame assembly **20**.

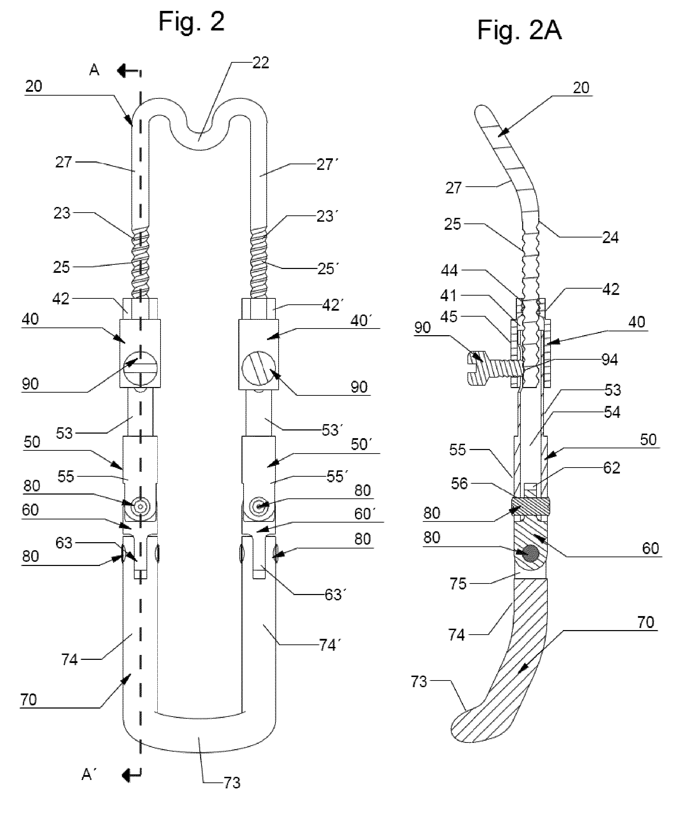

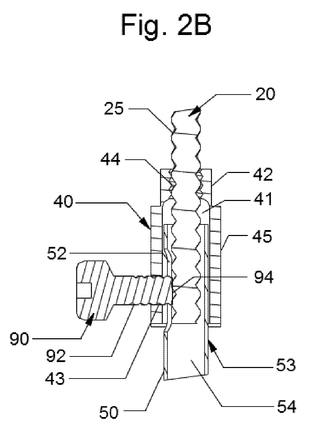

Tubular assemblies **50**; **50'** have an elongated shape with reduced portions **53**; **53'** and fork portions **55**; **55'**, adjacent to each other, as best seen in figures 1 and 3A. Longitudinal through bores **54; 54'** partially receive threaded bars **23; 23',** as best seen in figure 2A. Fork portions **55**; **55'** terminate with fork ends **57**; **57'.** Reduced portions **53**; **53'** include elongated guiding slots 52; 52' to allow ends **94; 94'** of shanks **92**; **92'** to pass herethrough and coact with threaded rod portions **25**; **25'** to secure or lock portions **45; 45'** in place relative to portions **25**; **25'**. The dimensions of slots **52; 52'** cooperate to guide shanks **92; 92'** and limiting the slidable movement of tubular portions **53**; **53'**. Fork portions **55**; **55'** terminate with fork ends **57**; **57'** include through holes **56**; **56'**.

Pivoting connector assemblies **60**; **60'** include ears **62**; **62'** each having through holes **61**; **61'** and ears **63**; **63'** each having through holes **64**; **64'**, as seen in figure 1. Cylindrical bodies **65**; **65'** provide the structure for the transition from ears **62**; **62'** to ears **63**; **63'**. Ears **62**; **62'** are disposed at 90 degrees with respect to ears **63**; **63'**. Ears **62**; **62'** are received within fork ends **57**; **57'** of fork portions **55**; **55'**, respectively. Through holes **61**; **61'** cooperatively coincide with through holes **56**; **56'** with cooperative dimensions to receive rivet pins **80; 80;** therethrough. Rivet pins **80**; **80'** are cylindrical and sized to extend beyond through holes **56**; **56'**. Once inside holes **56; 56'** are flattened to prevent their dislodgement. Fork portions **55**; **55'** and tubular assemblies **50; 50'** move to a maximum angle of approximately five degrees. The lateral movement of assemblies **50; 50'** with respect to connector assemblies **60**; **60'** is limited, in addition to the dimensional limitation of slots **52**; **52'** and the inner walls of longitudinal through opening **41**; **41'**. Ears **63; 63'** have similar through holes **64; 64'** that cooperate with through holes **72; 72; of** lower frame assembly **70'**.

Lower frame assembly **70** has elongated members **74; 74'** having fork connector terminations **75**; **75'** having fork connector ends **71**; **71'** with transversally disposed through holes **72**; **72'.** Assembly **70** has a substantially U-shape configuration. Members **74**; **74'** are connected through spacer member **73** that keeps the former in a rigid parallel and spaced apart relationship. Ends **71**; **71'** receive ears **63; 63'** and rivet pins **80; 80'** are passed through through holes **64; 64'** to allow tubular assemblies **50**; **50'** to pivot forward and rearwardly with respect to the plane of apparatus **10** as shown in figure 4.

When rivet pins **80** have cooperative dimensions so that when they are passed through through holes **56**; **56'**; **61**; **61'**; **63**; **63'**; **72** and **72'** they protrude slightly to permit a surgeon to hit them deforming the ends (riveting). This permits the joined members to pivot or rotate freely without being dislodged.

A physician will affix apparatus **10** to a patient's spinal bones using conventional means such as surgical wires and/or screws. With the present invention a patient can be provided with support and distention for his/her vertebrae of approximately five degrees per vertebra.

the foregoing description conveys the best understanding of the objectives and advantages of the present invention. Different embodiments may be made of the inventive concept of this invention. It is to be understood that all matter disclosed herein is to be interpreted merely as illustrative, and not in a limiting sense.

## Claims

1. An apparatus for treating spinal vertebrae, comprising:
A. a first U-shape frame assembly having first and second elongated bar members extending at a parallel and spaced apart relationship with respect to each other defining a first plane and said first and second elongated bar members having first and second threaded portions, respectively;
B. first and second elongated connector assemblies being at a parallel and spaced apart relationship with respect to each other and having third and fourth ends, said third ends including internal threaded openings mounted to said first and second threaded portions, respectively;
C. first and second parallel and spaced apart elongated tubular assemblies, having first and second longitudinal slots, and being telescopically and slidably mounted to said first and second bar members and partially housed by said first and second connector assemblies, respectively and further including first and second fastening members for mounting said first and second connector assemblies to said first and second threaded portions and passing through said slots;
D. first and second parallel and spaced apart and parallel elongated pivoting connector assemblies pivotally mounted to said first and second tubular assemblies, respectively, to permit a predetermined coplanar lateral movement of said first and second tubular assemblies with respect to said pivotally connector assemblies;
E. a second U-shape frame assembly having third and fourth elongated bar members extending at a parallel and spaced apart relationship with respect to each other defining a second plane and being pivotally mounted to said first and second elongated pivoting connector assemblies, respectively, to permit a predetermined movement of said second U-shaped frame member from a coplanar position with respect to the first plane tubular to different predetermined planes; and
F. means for mounting said apparatus to the spinal bones of a patient so that predetermined mechanical support and distention can be selectively and gradually applied to predetermined vertebrae as deemed necessary by the operating surgeon.

2. An apparatus for treating spinal vertebrae, comprising:
A. a first frame assembly having first and second elongated members each including first and second ends, and a first spacer member rigidly mounted to said first ends so that said first and second elongated members are kept at a parallel and spaced apart relationship with respect to each other, and said second ends each includes an external threaded portion;
B. first and second elongated connector assemblies each having a polygonal portion and a cylindrical portion, said polygonal portion including a coaxially disposed threaded through opening with a first internal diameter for cooperatively receiving said second ends, and each of said cylindrical portions including a coaxially disposed through opening, having a second internal diameter that is larger than said first internal diameter, connected to said threaded through opening, said polygonal portion being designed and adapted to receive a cooperating wrench for imparting a rotational force;
C. first and second elongated tubular assemblies each having third and fourth ends, said third ends being telescopically and slidably mounted within said coaxially disposed through openings including first and second fastening members to keep said first and second connector assembly at a predetermined relative position with respect to said first and second threaded portions and said fourth ends having each a first fork termination with a transversal first through hole therethrough;
D. first and second elongated pivoting connector assemblies each having fifth and sixth ends, said fifth ends each defining first ears with second through holes transversally disposed and said sixth ends defining second ears with third through holes transversally disposed and said second ears being disposed at 90 degrees with respect to said first ears;
E. first and second pins passed through said second through holes to keep said fifth ends and said first fork termination pivotally mounted to each other, so that said first frame assembly, elongated connector and tubular assemblies pivotally move laterally and coplanarly with respect to said pivoting connector assemblies in a first plane;
F. a second frame assembly having third and fourth elongated members each including seventh and eighth ends, and a second spacer member mounted to said seventh ends for keeping said third and fourth elongated members at a parallel spaced apart relationship with respect to each other, said eighth ends including a second fork termination with transversally disposed fourth throughholes;
G. third and fourth pins passed through said fourth through holes to keep said sixth ends and said second fork termination pivotally mounted to each other so that said second frame moves from a coplanar position with respect to said first plane to predetermined forward and rearward plane positions; and
H. means for mounting said apparatus to the spinal bones of a patient so that predetermined mechanical support and distention can be selectively and gradually applied to predetermined vertebrae as deemed necessary by the operating surgeon.
